# EUROPEAN PATENT SPECIFICATION

(11) **EP 1 315 724 B1**
(45) Date of publication and mention of the grant of the patent: **16.11.2005**
(21) Application number: 01963256.1
(22) Date of filing: 05.09.2001
(51) Int. Cl.: C07D 417/12, A61K 31/4439, A61P 3/10

(54) **A THIAZOLIDINEDIONE DERIVATIVE AND ITS USE AS ANTIDIABETIC**
EIN THIAZOLIDINDIONDERIVAT UND SEINE VERWENDUNG ALS ANTIDIABETIKUM
DERIVE DE THIAZOLIDINEDIONE ET SON EMPLOI EN TANT QU'ANTIDIABETIQUE

(30) Priority: 05.09.2000 GB 0021784
(43) Date of publication of application: 04.06.2003
(73) Proprietor: SMITHKLINE BEECHAM PLC, Brentford, Middlesex TW8 9GS (GB)
(72) Inventor: CRAIG, Andrew Simon, GlaxoSmithKline, Tonbridge, Kent TN11 9AN (GB); MILLAN, Michael, GlaxoSmithKline, Tonbridge, Kent TN11 9AN (AU)
(74) Representative: Rutter, Keith
(86) International application number: PCT/GB2001/003996
(87) International publication number: WO 2002/020520

(56) References cited:
- WO-A-94/05659
- WO-A-99/31093
- WO-A-99/31094
- WO-A-99/31095
- BERGE S M ET AL: "Pharmaceutical Salts" JOURNAL OF PHARMACEUTICAL SCIENCES, vol. 66, no. 1, January 1977 (1977-01), pages 1-19, XP002152518

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 of EP 0,306,228 is 5-[4-[2-(N-methyl-N-(2-pyridyl)amino)ethoxy]benzyl]thiazolidine-2,4-dione (hereinafter also referred to as "Compound (I)").

International Patent Application, Publication Number WO94/05659 discloses certain salts of the compounds of EP 0,306,228. The preferred salt of WO94/05659 is the maleic acid salt.

WO 99/331093, WO 99/31094 and WO 99/31095 (all SmithKline Beecham PLC) each disclose certain hydrates of the maleic acid salt of 5-[4-[2-(N-methyl-N-(2-pyridyl)amino)-ethoxy]benzyl]thiazolidine-2,4-dione.

It has now been discovered that Compound (I) forms a novel ethanesulfonate salt (hereinafter also referred to as the "Ethanesulfonate") that is particularly stable and hence is suitable for bulk preparation and handling. The Ethanesulfonate also shows particularly good aqueous solubility and possesses good bulk flow properties. The Ethanesulfonate is therefore surprisingly amenable to large scale pharmaceutical processing and especially to large scale miling. The novel salt can be prepared by an efficient, economic and reproducible process particularly suited to large-scale preparation

The novel Ethanesulfonate also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione ethanesulfonate salt or a solvate thereof.

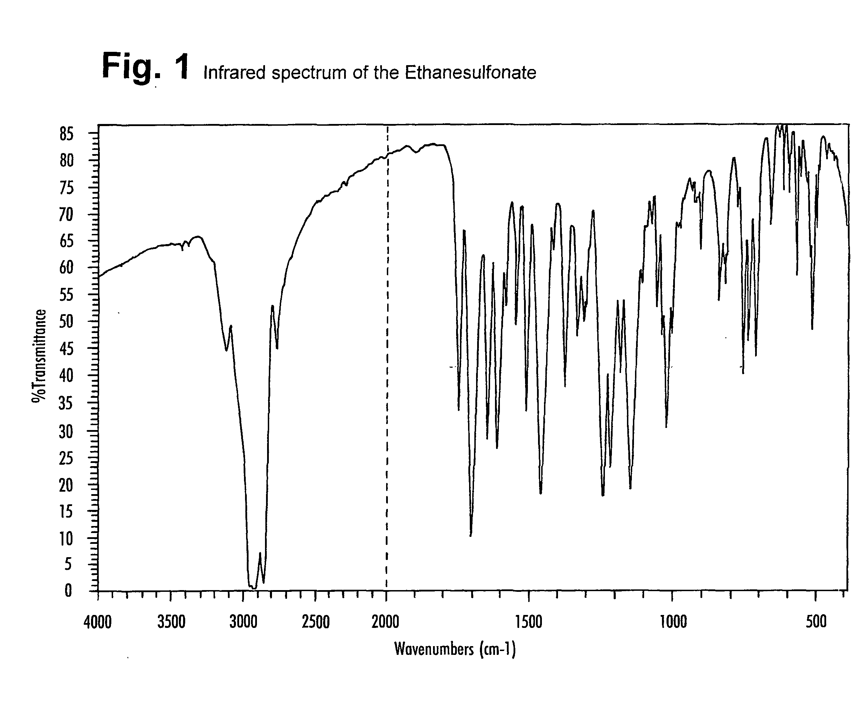

In one favoured aspect, the Ethanesulfonate provides an infrared spectrum substantially in accordance with Figure 1.

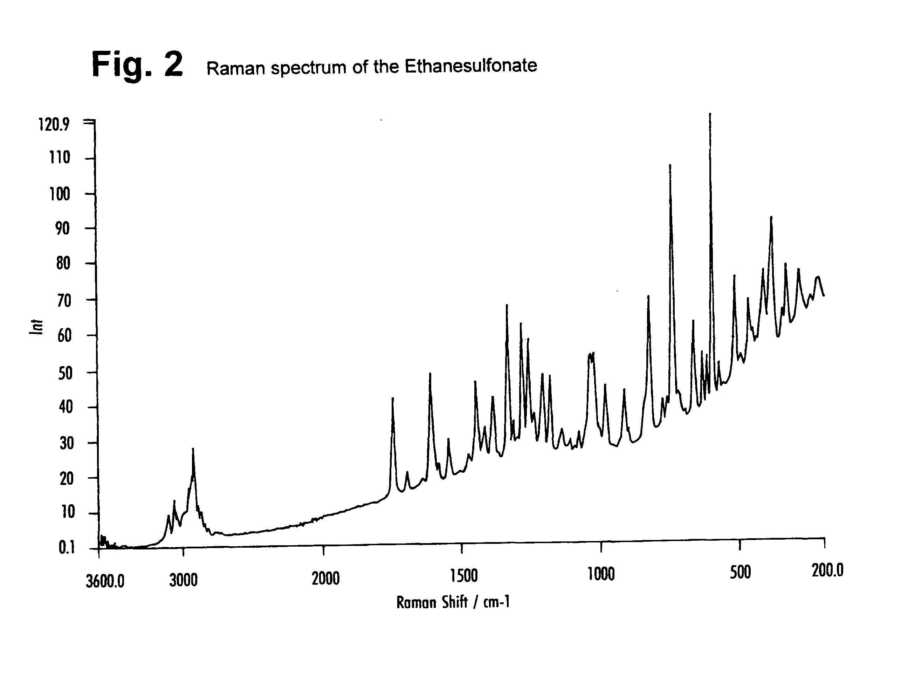

In one favoured aspect, the Ethanesulfonate provides a Raman spectrum substantially in accordance with Figure 2.

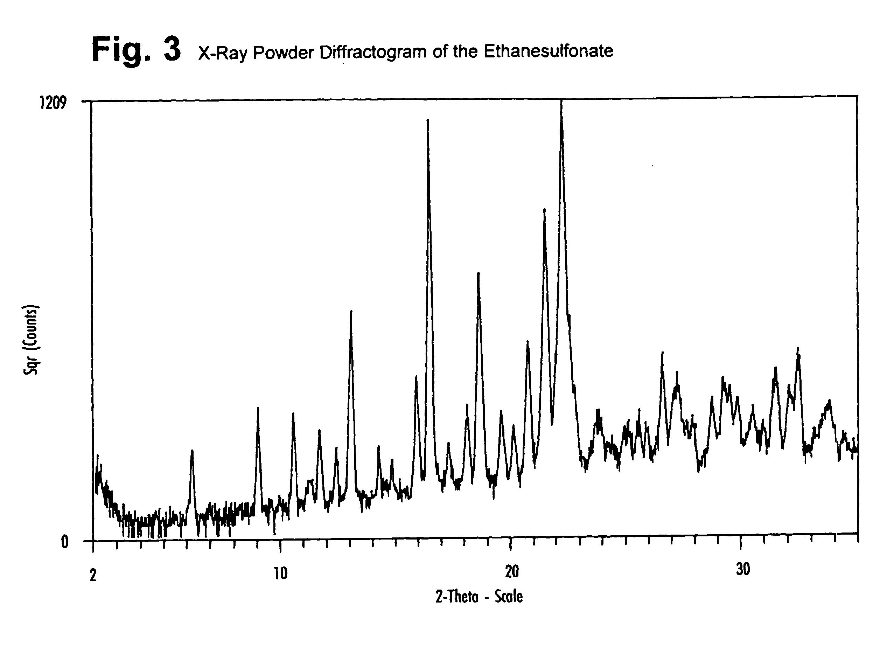

In one favoured aspect, the Ethanesulfonate provides an X-Ray powder diffraction pattern (XRPD) substantially in accordance with Table 1 or Figure 3.

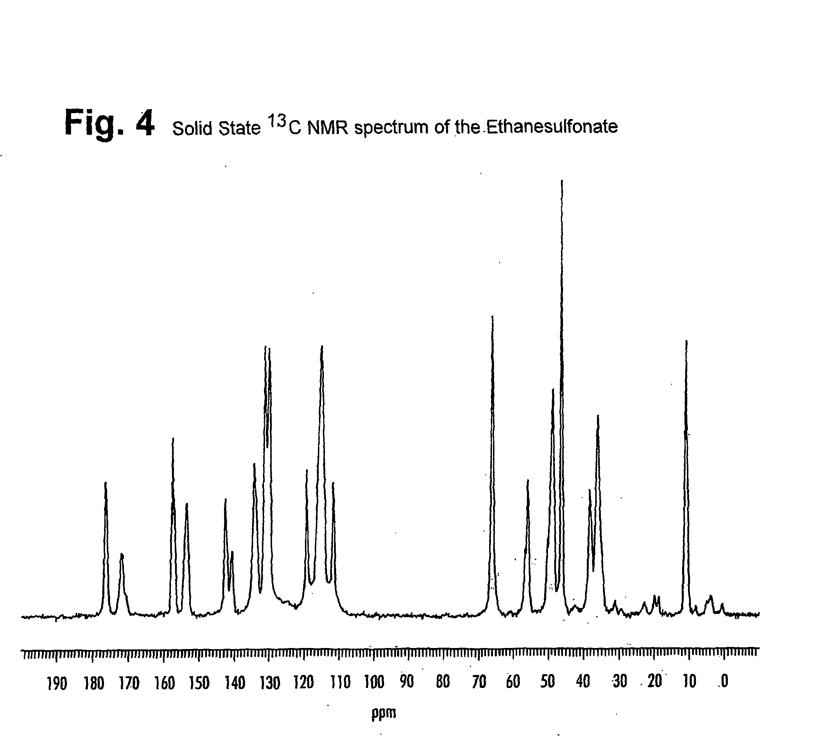

In one favoured aspect, the Ethansulfonate provides a Solid State ¹³C NMR spectrum substantially in accordance with Figure 4.

In a preferred aspect, the invention provides 5-[4-[2-(N-methyl-N-(2-pyridyl)amino)ethoxy]benzyl]thiazolidine-2,4-dione, ethanesulfonate salt, characterised in that it provides:
(i) an infrared spectrum substantially in accordance with Figure 1; and
(ii) a Raman spectrum substantially in accordance with Figure 2; and
(iii) an X-Ray powder diffraction pattern (XRPD) substantially in accordance with Table 1 or Figure 3; and
(iv) a Solid State ¹³C NMR spectrum substantially in accordance with Figure 4.

The present invention encompasses the Ethanesulfonate or a solvate thereof isolated in pure form or when admixed with other materials.

Thus in one aspect there is provided the Ethanesulfonate or a solvate thereof in isolated form.

In a further aspect there is provided the Ethanesulfonate or solvate thereof in a purified form.

In yet a further aspect there is provided the Ethanesulfonate or solvate thereof in crystalline form.

Also, the invention provides the Ethanesulfonate or solvate thereof in a solid pharmaceutically acceptable form, such as a solid dosage form, especially when adapted for oral administration.

Moreover, the invention also provides the Ethanesulfonate or solvate thereof in a pharmaceutically acceptable form, especially in bulk form, such form being particularly capable of being milled. The invention therefor also provides the Ethanesulfonate or solvate thereof in a milled form.

Furthermore, the invention provides the Ethanesulfonate or solvate thereof in a pharmaceutically acceptable form, especially in bulk form, such form having good flow properties, especially good bulk flow properties.

A suitable solvate is a hydrate.

The invention also provides a process for preparing the Ethanesulfonate or a solvate thereof, characterised in that 5-[4-[2-(N-methyl-N-(2-pyridyl)amino)ethoxy]benzyl]thiazolidine-2,4-dione (Compound (I)), or a salt thereof, preferably dispersed or dissolved in a suitable solvent, is reacted with a source of ethanesulfonate ion and thereafter, if required, a solvate of the resulting Ethanesulfonate is prepared; and the Ethanesulfonate or a solvate thereof is recovered.

Suitably, Compound (I) is used in the reaction, especially in a protonated form.

Suitably, a salt of Compound (I) is used in the reaction..

Compound (I) is preferably present in a protonated form.

A suitable reaction solvent is an alkanol, for example propan-2-ol, or a hydrocarbon, such as toluene, a ketone, such as acetone, an ester, such as ethyl acetate, an ether such as tetrahydrofuran, a nitrile such as acetonitrile, or a halogenated hydrocarbon such as dichloromethane or water, or an organic acid such as acetic acid; or a mixture thereof.

Conveniently, the source of Ethanesulfonate ion is ethanesulfonic acid. The ethanesulfonic acid may be added per se or in solution, for example in a lower alcohol such as methanol, ethanol, or propan-2-ol, or a mixture of solvents. An alternative source of Ethanesulfonate ion is provided by a base salt of ethanesulfonic acid for example ammonium ethanesulfonate, or the ethanesulfonic acid salt of an amine, for example ethylamine or diethylamine.

The concentration of Compound (I) is preferably in the range 2 to 25% weight/volume, more preferably in the range 5 to 20%. The concentration of ethanesulfonic acid solutions are preferably in the range of 10 to 100 % weight/volume.

The reaction is usually carried out at ambient temperature or at an elevated temperature, for example 50 - 60°C, although any convenient temperature that provides the required product may be employed.

Solvates, such as hydrates, of the Ethanesulfonate are prepared according to conventional procedures.

Recovery of the required compound generally comprises crystallisation from an appropriate solvent or mixture of solvents, conveniently the reaction solvent, usually assisted by cooling. For example, the Ethanesulfonate may be crystallised from an alcohol such as propan-2-ol. An improved yield of the salt can be obtained by evaporation of some or all of the solvent or by crystallisation at elevated temperature followed by controlled cooling, optionally in stages. Careful control of precipitation temperature and seeding may be used to improve the reproducibility of the product form.

Crystallisation can also be initiated by seeding with crystals of the Ethanesulfonate or a solvate thereof but this is not essential.

Compound (I) is prepared according to known procedures, such as those disclosed in EP 0,306,228 and WO94/05659. The disclosures of EP 0,306,228 and WO94/05659 are incorporated herein by reference.

Ethanesulfonic acid is a commercially available compound.

When used herein in respect of certain compounds the term "good flow properties" is suitably characterised by the said compound having a Hausner ratio of less than or equal to 1.5, especially of less than or equal to 1.25.

"Hausner ratio" is an art accepted term.

When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the Ethanesulfonate or a solvate thereof for use as an active therapeutic substance.

More particularly, the present invention provides the Ethanesulfonate or a solvate thereof for use in the treatment and/or prophylaxis of diabetes mellitus, conditions associated with diabetes mellitus and certain complications thereof.

The Ethanesulfonate or a solvate thereof may be administered per se or, preferably, as a pharmaceutical composition also comprising a pharmaceutically acceptable carrier. Suitable methods for formulating the Ethanesulfonate or a solvate thereof are generally those disclosed for Compound (I) in the above mentioned publications.

Accordingly, the present invention also provides a pharmaceutical composition comprising the Ethanesulfonate or a solvate thereof and a pharmaceutically acceptable carrier therefor.

The Ethanesulfonate or a solvate thereof is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulfate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press) and Harry's Cosmeticology (Leonard Hill Books).

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of Ethanesulfonate or a solvate thereof to a human or non-human mammal in need thereof.

Conveniently, the active ingredient may be administered as a pharmaceutical composition hereinbefore defined, and this forms a particular aspect of the present invention.

In a further aspect the present invention provides the use of Ethanesulfonate or a solvate thereof for the manufacture of a medicament for the treatment and/or prophylaxis of diabetes mellitus; conditions associated with diabetes mellitus and certain complications thereof.

In the treatment and/or prophylaxis of diabetes mellitus, conditions associated with diabetes mellitus and certain complications thereof the Ethanesulfonate or a solvate thereof may be taken in amounts so as to provide Compound (I) in suitable doses, such as those disclosed in EP 0,306,228, WO94/05659 or WO98/55122.

No adverse toxicological effects are indicated in the above mentioned treatments for the compounds of the invention.

The following examples illustrate the invention but do not limit it in any way.

### EXAMPLES

### Example 1 5-[4-[2-(N-methyl-N-(2-pyridyl)amino)ethoxy]benzyl]thiazolidine-2,4-dione ethanesulfonate

Ethanesulfonic acid (0.68 mL) was added to a stirred suspension of 5-[4-[2-(N-methyl-N-(2-pyridyl)amino)ethoxy]benzyl]thiazolidine-2,4-dione (3.0 g) in propan-2-ol (90 mL) at 50°C. The stirred mixture was maintained at 50°C until a clear solution was observed and was then cooled to 21 °C over approximately 1 hour. The white precipitate was collected by filtration, washed with propan-2-ol (10 mL), and dried for 16 hours under vacuum to give 5-[4-[2-(N-methyl-N-(2-pyridyl)amino)ethoxy]benzyl]thiazolidine-2,4-dione ethanesulfonate as a white crystalline solid (3.3 g).

¹H-NMR (d₆-DMSO): consistent with 5-[4-[2-(N-methyl-N-(2-pyridyl)amino)ethoxy]benzyl]thiazolidine-2,4-dione ethanesulfonate.

### Example 2 5-[4-[2-(N-methyl-N-(2-pyridyl)amino)ethoxy]benzyl]thiazolidine-2,4-dione ethanesulfonate

A stirred suspension of 5-[4-[2-(N-methyl-N-(2-pyridyl)amino)ethoxy]benzyl] thiazolidine-2,4-dione (20.0 g) in propan-2-ol (200 mL) was heated to 60°C before ethanesulfonic acid (4.57 mL) was added. The mixture was held at 60°C until a clear solution was observed. The stirred mixture was then cooled to 21 °C over approximately one hour. The white product was collected by filtration, washed with propan-2-ol (2 x 30 mL) and dried under vacuum for 16 hours to give 5-[4-[2-(N-methyl-N-(2-pyridyl)amino)ethoxy]benzyl]thiazolidine-2,4-dione ethanesulfonate as a white crystalline solid (24.0 g).

### Characterising data recorded for the product of Example 1

The infrared absorption spectrum of a mineral oil dispersion of the product was obtained using a Nicolet 710 FT-IR spectrometer at 2 cm⁻¹ resolution (Figure 1). Data were digitised at 1 cm⁻¹ intervals. Bands were observed at: 3117, 2772, 1750, 1704, 1647, 1616, 1586, 1549, 1514, 1458, 1419, 1335, 1313, 1241, 1218, 1185, 1146, 1078, 1059, 1041, 1023, 1006, 907, 841, 820, 779, 758, 740, 715, 664, 576, 520, 506 cm⁻¹.

The infrared spectrum of the solid product was recorded using a Perkin-Elmer Spectrum One FT-IR spectrometer fitted with a universal ATR accessory. Bands were observed at: 3122, 3000, 2771, 1749, 1699, 1647, 1612, 1587, 1550, 1514, 1480, 1458, 1419, 1386, 1334, 1313, 1240, 1216, 1185, 1141, 1079, 1058, 1021, 1006, 907, 840, 820, 779, 757, 739, 714, 663 cm⁻¹.

The Raman spectrum of the Ethanesulfonate (Figure 2) was recorded with the sample in a glass vial using a Perkin-Elmer 2000R FT-Raman spectrometer, at 4 cm⁻¹ resolution with excitation from a Nd:YAG laser (1064 nm) with a power output of 400 mW. Bands were observed at: 3109, 3067, 2928, 1749, 1699, 1613, 1548, 1451, 1420, 1391, 1337, 1318, 1287, 1263, 1211, 1185, 1143, 1082, 1042, 987, 919, 828, 780, 744, 667, 636, 620, 601, 576, 520, 470, 417, 387, 337, 292, 225 cm⁻¹.

The X-Ray Powder Diffractogram pattern of the product (Figure 3) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 1.

**Table 1**

| **Angle** | **Rel. Intensity** |
|---|---|
| **2-Theta°** | **%** |
| 6.2 | 4.1 |
| 9.1 | 9 |
| 10.6 | 8.1 |
| 11.3 | 1.7 |
| 11.7 | 6 |
| 12.4 | 4.3 |
| 13.1 | 27 |
| 14.3 | 4.3 |
| 14.9 | 3.2 |
| 16.0 | 13.6 |
| 16.6 | 92.1 |
| 17.4 | 4.5 |
| 18.2 | 9.2 |
| 18.7 | 36.7 |
| 19.7 | 8.4 |
| 20.2 | 6.5 |
| 20.9 | 19.9 |
| 21.6 | 56.8 |
| 22.4 | 100 |
| 23.8 | 7.1 |
| 24.0 | 7.4 |
| 25.2 | 6.6 |
| 25.6 | 8.3 |
| 26.0 | 5.8 |
| 26.7 | 17.5 |
| 27.3 | 13.8 |
| 28.0 | 7.6 |
| 28.9 | 9.8 |
| 29.4 | 12.2 |
| 29.6 | 11.7 |
| 29.9 | 9.9 |
| 30.6 | 7.8 |
| 31.1 | 6.8 |
| 31.6 | 14.2 |
| 32.2 | 10.7 |
| 32.6 | 17.9 |
| 33.8 | 9.1 |
| 34.5 | 5.2 |

The solid-state NMR spectrum of the product (Figure 4) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 11.1, 36.1, 38.5, 46.5, 48.9, 56.1, 66.2, 111.7, 114.9, 119.2, 129.9, 131.1, 134.1, 140.5. 142.4, 153.4, 157.2, 171.8, 176.1 ppm.

### Properties of the Ethanesulfonate, recorded for the product of Example 2

### Solid State Stability of the Ethanesulfonate

The solid state stability of the drug substance was determined by storing approximately 1.0 g of the material in a glass bottle at a) 40°C / 75% Relative Humidity (RH), open exposure, for 1 month and b) at 50°C, closed, for 1 month. The material was assayed by HPLC for final content and degradation products in both cases.
a) 40°C / 75% RH: No significant degradation observed (HPLC assay 99% initial).
b) 50°C: No significant degradation observed (HPLC assay 100% initial).

### Solubility of the Ethanesulfonate

The solubility of the material was determined by adding water in aliquots from 1 to 1000ml to approximately 100mg of drug substance until the powder had dissolved. The visual solubility was confirmed by an HPLC assay of a saturated solution.
Solubility: > 100 mg/ml.

### Flow Properties of the Ethanesulfonate:

The ratio between the bulk density and the tapped bulk density (Hausner Ratio) of the Ethanesulfonate was determined using standard methods ("Pharmaceutics - The Science of Dosage Form Design", editor M. Aulton, 1988, published by:Churchill Livingstone).
Hausner Ratio: 1.1.

## Claims

1. 5-[4-[2-(N-methyl-N-(2-pyridyl)amino)ethoxy]benzyl]thiazolidine-2,4-dione ethanesulfonate salt, or a solvate thereof.

2. A compound according to claim 1 in purified form.

3. A compound according to claim 1 in a solid dosage form.

4. A compound according to claim 1 in a pharmaceutically acceptable form capable of being milled, or in milled form.

5. A compound according to claim 1 in a pharmaceutically acceptable form and having good flow properties.

6. A process for preparing the ethanesulfonate or a solvate thereof, **characterised in that** 5-[4-[2-(N-methyl-N-(2-pyridyl)amino)ethoxy]benzyl]thiazolidine-2,4-dione or a salt thereof, preferably dispersed or dissolved in a suitable solvent, is reacted with a source of ethanesulfonate ion and thereafter, if required, a solvate of the resulting Ethanesulfonate is prepared; and the Ethanesulfonate or a solvate thereof is recovered.

7. A pharmaceutical composition comprising 5-[4-[2-(N-methyl-N-(2-pyridyl)amino)ethoxy]benzyl]thiazolidine-2,4-dione ethanesulfonate or a solvate thereof and a pharmaceutically acceptable carrier therefor.

8. A compound 5-[4-[2-(N-methyl-N-(2-pyridyl)amino)ethoxy]benzyl]thiazolidine-2,4-dione ethanesulfonate or a solvate thereof for use as an active therapeutic substance.

9. A use of 5-[4-[2-(N-methyl-N-(2-pyridyl)amino)ethoxy]benzyl]thiazolidine-2,4-dione ethanesulfonate or a solvate thereof, for the manufacture of a medicament for the treatment and/or prophylaxis of diabetes mellitus, conditions associated with diabetes mellitus and certain complications thereof.

## Patentansprüche

1. 5-[4-[2-(N-Methyl-N-(2-pyridyl)amino)ethoxy]benzyl]thiazolidin-2,4-dionethansulfonatsalz, oder ein Solvat davon.

2. Verbindung gemäß Anspruch 1 in gereinigter Form.

3. Verbindung gemäß Anspruch 1 in einer festen Dosierungsform.

4. Verbindung gemäß Anspruch 1 in einer pharmazeutisch verträglichen Form, welche geeignet ist, gemahlen zu werden, oder in gemahlener Form.

5. Verbindung gemäß Anspruch 1 in einer pharmazeutisch verträglichen Form und mit guten Fließeigenschaften.

6. Verfahren zur Herstellung des Ethansulfonats oder einem Solvat davon, **dadurch gekennzeichnet, dass** 5-[4-[2-(N-Methyl-N-(2-pyridyl)amino)ethoxy]benzyl]thiazolidin-2,4-dion oder ein Salz davon, bevorzugt dispergiert oder in einem geeigneten Lösungsmittel gelöst, mit einer Quelle für Ethansulfonationen umgesetzt wird und danach, falls benötigt, ein Solvat des resultierenden Ethansulfonats hergestellt wird; und das Ethansulfonat oder ein Solvat davon gewonnen wird.

7. Arzneimittel, umfassend 5-[4-[2-(N-Methyl-N-(2-pyridyl)amino)ethoxy]benzyl]thiazolidin-2,4-dion-ethansulfonat oder ein Solvat davon und einen pharmazeutisch verträglichen Träger dafür.

8. Verbindung 5-[4-[2-(N-Methyl-N-(2-pyridyl)amino)ethoxy]benzyl]thiazolidin-2,4-dionethansulfonat oder ein Solvat davon zur Verwendung als therapeutischer Wirkstoff.

9. Verwendung von 5-[4-[2-(N-Methyl-N-(2-pyridyl)amino)ethoxy]benzyl]thiazolidin-2,4-dion-ethansulfonat oder einem Solvat davon zur Herstellung eines Medikaments zur Behandlung und/oder Prophylaxe von Diabetes Mellitus, Zuständen, welche im Zusammenhang mit Diabetes Mellitus stehen, und bestimmten Komplikationen davon.

## Revendications

1. Sel éthanesulfonate de 5-[4-[2-(N-méthyl-N-(2-pyridyl)amino)éthoxy)benzyl]thiazolidine-2,4-dione, ou un de ses produits de solvatation.

2. Composé suivant la revendication 1, sous forme purifiée.

3. Composé suivant la revendication 1, sous une forme posologique solide.

4. Composé suivant la revendication 1, sous une forme pharmaceutiquement acceptable pouvant être broyée, ou sous forme broyée.

5. Composé suivant la revendication 1, sous une forme pharmaceutiquement acceptable et ayant de bonnes propriétés d'écoulement.

6. Procédé pour la préparation de l'éthanesulfonate ou d'un de ses produits de solvatation, **caractérisé en ce que** de la 5-[4-[2-(N-méthyl-N-(2-pyridyl)amino)éthoxy]-benzyl]thiazolidine-2,4-dione ou un de ses sels, de préférence à l'état dispersé ou dissous dans un solvant convenable, est amené à réagir avec une source d'ion éthanesulfonate et, ensuite, si besoin, un produit de solvatation de l'éthanesulfonate résultant est préparé ; et l'éthanesulfonate ou un de ses produits de solvatation est recueilli.

7. Composition pharmaceutique comprenant de l'éthanesulfonate de 5-[4-[2-(N-méthyl-N-(2-pyridyl)amino)-éthoxy]benzyl)thiazolidine-2,4-dione ou un de ses produits de solvatation et un support pharmaceutiquement acceptable à cette fin.

8. Composé éthanesulfonate de 5-[4-[2-(N-méthyl-N-(2-pyridyl)amino)éthoxy]benzyl]thiazolidine-2,4-dione ou un de ses produits de solvatation destiné à être utilisé comme substance thérapeutique active.

9. Utilisation d'éthanesulfonate de 5-[4-[2-(N-méthyl-N-(2-pyridyl)amino)éthoxy]benzyl]thiazolidine-2,4-dione ou d'un de ses produits de solvatation pour la production d'un médicament destiné au traitement et/ou à la prophylaxie du diabète sucré, d'affections associées au diabète sucré et de certaines de leurs complications.
